# FASCICULE DE BREVET EUROPEEN

(11) **EP 1 079 789 B1**
(45) Date de publication et mention de la délivrance du brevet: **22.10.2003**
(21) Numéro de dépôt: 00910989.3
(22) Date de dépôt: 17.03.2000
(51) Int. Cl.: A61J 1/06, A61M 5/00

(54) **AMPOULE POUR LE CONDITIONNEMENT D'UN LIQUIDE A USAGE MEDICAL**
AMPULLE ZUR AUFNAHME EINER MEDIZINISCHEN FLÜSSIGKEIT
AMPOULE CONTAINING A LIQUID FOR MEDICAL PURPOSES

(30) Priorité: 25.03.1999 FR 9903901
(43) Date de publication de la demande: 07.03.2001
(73) Titulaire: Frezza, Pierre, 69390 Charly (FR)
(72) Inventeur: Frezza, Pierre, 69390 Charly (FR)
(74) Mandataire: Maureau, Philippe
(86) Numéro de dépôt international: FR0000670
(87) Numéro de publication internationale: WO00057834

(56) Documents cités:
- EP-A- 0 897 708
- WO-A-96/00556
- US-A- 2 400 722
- US-A- 3 563 373
- US-A- 3 696 579
- US-A- 4 390 016
- US-A- 4 401 432

## Description

Une ampoule pour le conditionnement d'un liquide à usage médical est un petit réservoir dont le volume varie de quelques millilitres à quelques dizaines de millilitres, destiné à contenir de manière étanche et stérile un principe actif, ou un solvant (NaCl à 0,9 %, Glucose à 5 %). Le réservoir est réalisé en un matériau biocompatible avec son contenu et de qualité pharmaceutique. Le matériau constitutif du réservoir peut être du verre ou une matière synthétique. Les ampoules en verre sont destinées à être progressivement remplacées par des ampoules en matière synthétique, qui sont plus pratiques d'utilisation.

Le contenu d'une ampoule est généralement utilisé pour la dilution de principes actifs ou pour la reconstitution de médicaments en poudre.

La mise en oeuvre d'une ampoule dans le cadre d'une reconstitution est la suivante :
- l'opérateur désinfecte la zone d'ouverture de l'ampoule, puis casse l'élément ruptible d'accès au contenu de celle-ci.
- l'opérateur retire une aiguille hypodermique stérile de son conditionnement, fixe l'aiguille sur une seringue, et retire la gaine rigide formant protège-aiguille.
- l'opérateur prélève par aspiration à l'intérieur de l'ampoule un volume donné de liquide, ce volume pouvant être contrôlé grâce aux graduations de la seringue.
- le liquide étant prélevé dans la seringue, l'opérateur transfère celui-ci dans un flacon contenant la poudre ou le lyophilisat à diluer. Le flacon est operculé de manière étanche par un bouchon en élastomère perforable par l'aiguille de la seringue.
- après homogénéisation, la solution est de nouveau aspirée par la seringue.
- le produit, ainsi préparé est stocké dans la seringue munie de son aiguille, peut ensuite être transféré dans une poche ou un flacon de perfusion, toujours au moyen de l'aiguille en perforant une membrane étanche prévue à cet effet sur la poche ou sur le flacon de perfusion.
- après utilisation, l'aiguille est remise en place à l'intérieur du protège-aiguille pour être jetée sans risque de piqûre accidentelle.

Cette technique nécessite, pour la mise en oeuvre du contenu d'une ampoule, une pluralité d'éléments : aiguille logée à l'intérieur d'un protecteur et conditionnée dans un emballage stérile, seringue, et ampoule contenant le liquide. Le nombre d'opérations à effectuer est important. Il convient de réaliser l'ouverture du conditionnement de l'aiguille, de fixer celle-ci sur la seringue, d'enlever le protecteur d'aiguille, de désinfecter l'ampoule, de casser l'ampoule avec le risque de génération de particules pouvant tomber à l'intérieur du liquide contenu dans celle-ci, et d'introduire l'aiguille dans un trou de faible diamètre.

Le document D1 (US 3,563,373 - Paulson) décrit un dispositif à usage médical constitué de cartouches préremplies, assemblées et emballées pour mélanger des composés incompatibles au moment de l'injection.

Le dispositif de D1 comprend deux chambres séparées par une paroi ruptible ou perforable par une aiguille au moment de l'injection. Après perforation, l'aiguille n'est pas protégée d'un contact avec les parois du récipient alentour.

Il en résulte un prix de revient élevé tant au niveau du dispositif lui-même que de sa mise en oeuvre.

Le but de l'invention est de fournir une ampoule pour le conditionnement d'un liquide à usage médical, qui soit d'un prix de revient économique, et dont la mise en oeuvre soit simple, sûre et rapide pour l'utilisateur.

A cet effet, l'ampoule qu'elle concerne comporte un corps tubulaire en matière synthétique présentant deux chambres isolées de manière étanche par une membrane perforable ou une paroi amovible ou ruptible, dont l'une contient le liquide et dont l'autre contient une aiguille hypodermique dont la partie pointue est tournée vers la membrane perforable ou la paroi amovible ou ruptible, et dont l'autre extrémité est munie de moyens de raccordement à un corps de seringue.

La chambre contenant l'aiguille comprend un tube formant porte-aiguille fermé par la membrane ou la paroi amovible ou ruptible et présentant, à son extrémité débouchant dans la chambre contenant l'aiguille, un jonc annulaire d'étanchéité. L'aiguille est donc conditionnée dans la chambre prévue à cet effet, la partie formant aiguille proprement dite étant à l'intérieur du tube, et l'extrémité munie de moyens de raccordement à un corps de seringue étant à l'extérieur du tube. En pratique, l'opérateur ouvre la chambre contenant l'aiguille, fixe sur celle-ci le corps d'une seringue, et exerce une poussée sur l'aiguille pour perforer la membrane de mise en communication avec la chambre contenant le liquide, ou rompt la paroi ruptible correspondant à cette membrane. Le jonc annulaire d'étanchéité assure la fermeture étanche de la chambre contenant le liquide. L'opérateur peut alors aspirer à l'aide de la seringue la quantité de liquide souhaitée, ce qui est favorisé par le fait que le corps tubulaire en matière synthétique est déformable. Après utilisation du contenu de la seringue de façon traditionnelle, l'opérateur peut replacer l'aiguille à l'intérieur du tube formant porte-aiguille, ce qui assure la protection de celle-ci lors de son évacuation en vue d'une destruction.

Suivant l'invention, la longueur du tube destiné à recevoir l'aiguille est au moins égale à celle de l'aiguille, la membrane ou la paroi amovible ou ruptible étant située à proximité de l'extrémité du tube tournée vers la chambre contenant le liquide et le jonc situé à proximité de l'autre extrémité du tube étant destiné à assurer l'étanchéité autour des moyens de raccordement de l'aiguille à un corps de seringue.

En outre, la distance entre l'extrémité libre du tube et la membrane ou la paroi amovible ou ruptible est au moins égale à la course de l'aiguille entre une position de stockage et une position de prélèvement du liquide dans laquelle l'extrémité pointue de l'aiguille traverse la membrane ou la paroi amovible ou ruptible et les moyens de raccordement de l'aiguille sont enserrés dans le jonc d'étanchéité. Dans ces conditions, l'aiguille ne dépasse pas de l'extrémité du tube après perforation de la membrane ou rupture de la paroi amovible, de telle sorte que les risques de perforation accidentelle de la paroi de l'ampoule par la pointe de l'aiguille sont supprimés. Le mouvement de translation de l'aiguille est limité par un épaulement que comporte celle-ci dans la zone de raccordement entre l'aiguille hypodermique elle-même et les moyens de raccordement de l'aiguille, par exemple de type Luer femelle, au corps d'une seringue.

Avantageusement, le tube comprend, à proximité du jonc d'étanchéité, des moyens de blocage en rotation de l'aiguille, tels que nervures ou bossages, destinés à coopérer avec des moyens complémentaires que comporte l'aiguille. Cette caractéristique facilite la fixation de l'aiguille sur le corps de seringue en empêchant l'aiguille de tourner en même temps que la seringue.

Selon une forme préférée d'exécution de cette ampoule, le tube destiné à recevoir l'aiguille est solidaire d'une paroi transversale dont la périphérie est solidaire de la paroi intérieure du corps tubulaire. Le tube forme avantageusement une seule pièce avec la paroi intérieure du corps tubulaire.

Selon une première forme d'exécution de cette ampoule, le corps tubulaire, la paroi séparant les deux chambres et le porte-aiguille sont réalisés en une pièce de matière synthétique obtenue par moulage.

Dans ce cas, par exemple, la chambre contenant le liquide est obturée par une soudure thermique, après remplissage en liquide et aplatissement du corps, et la chambre contenant l'aiguille, dont le bord est limité par une collerette, est fermée par un opercule, tel qu'un film perméable à la vapeur, fixé par soudage à chaud, ou par un obturateur en matière synthétique fixé avec étanchéité par vissage ou encliquetage. La fermeture, par un film perméable à la vapeur, permet de réaliser une stérilisation de la chambre contenant l'aiguille.

Suivant une autre caractéristique de l'invention, d'une part, la paroi séparant les deux chambres et le porte-aiguille sont réalisés en une pièce de matière synthétique obtenue par moulage et, d'autre part, le corps tubulaire est constitué par un tube de matière synthétique extrudé ou réalisé à partir de deux films de matière synthétique éventuellement thermoformés, fixé par soudage sur la paroi séparant les deux chambres, et dont les deux extrémités sont fermées par des soudures thermiques après formation des deux chambres, remplissage de la chambre contenant le liquide et mise en place de l'aiguille dans l'autre chambre.

Il est, dans un tel cas, possible de réaliser le corps tubulaire en un matériau beaucoup plus souple que la paroi séparant les deux chambres et le porte-aiguille, et de donner aux extrémités des deux chambres, et notamment à l'extrémité de la chambre contenant le liquide, une forme, par exemple, en V permettant de limiter au maximum le volume résiduel de liquide à l'intérieur de cette chambre.

De toute façon, l'invention sera bien comprise, à l'aide de la description qui suit, en référence au dessin schématique annexé représentant, à titre d'exemples non limitatifs, plusieurs formes d'exécution de cette ampoule :
Figure 1 est une vue en coupe transversale et en position éclatée des différents éléments constitutifs de l'ampoule ;
Figure 2 est une vue en coupe longitudinale de la même ampoule en position de fermeture ;
Figures 3 à 5 sont trois vues en coupe représentant trois phases d'utilisation de cette ampoule ;
Figure 6 est une vue en coupe transversale et en position remplie d'une autre ampoule.

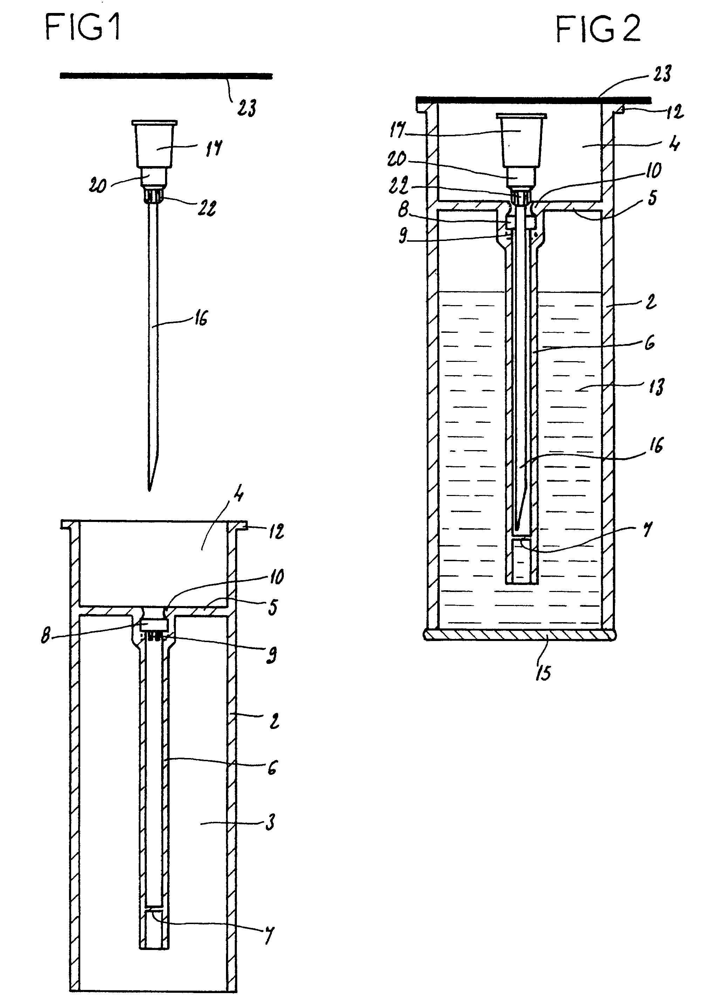

L'ampoule représentée à la figure 1 comprend un corps tubulaire cylindrique 2 en matière synthétique, présentant deux chambres, une chambre inférieure 3 et une chambre supérieure 4, séparées l'une de l'autre de façon étanche par une cloison 5 en forme de disque. A partir de cette cloison 5 s'étend au centre du corps tubulaire, et à l'intérieur de la chambre 3, un tube 6 débouchant dans la chambre 4, et présentant, à proximité de son extrémité située dans la chambre 3, une membrane 7 ou une paroi ruptible ou amovible. L'intérieur du tube 6 communique avec la chambre 4. L'extrémité du tube 6 située du côté de la chambre 4 comprend une partie élargie 8 dans le fond de laquelle est disposé un système de rainures/nervures longitudinales 9. Cette partie élargie 8 est raccordée à la chambre 4 par un jonc annulaire 10. Le corps tubulaire 2 comporte une extrémité située du côté de la chambre 4, entourée par une collerette 12.

Dans la forme d'exécution représentée à la figure 1, le cylindre 2, la cloison 5 et le tube 6 sont constitués par une pièce unique en matière synthétique obtenue par moulage. Cette pièce est transparente, et la paroi du corps tubulaire possède une bonne souplesse permettant sa déformation. Après remplissage de la chambre 3 par un liquide 13, celle-ci est obturée par une soudure thermique 15, après aplatissement du corps. La chambre 4 et le tube 6 sont destinés à servir de logement à une aiguille hypodermique 16 équipée d'un dispositif de raccordement 17, de type Luer femelle, à une seringue 18 dont l'orifice de sortie est équipé d'un raccord Luer mâle 19. La zone de raccordement 17 de l'aiguille 16 est prolongée du côté de l'aiguille par une partie 20 destinée à être engagée avec étanchéité dans le jonc annulaire 10, ainsi que par un système de rainures/nervures 22 destiné à coopérer avec le système de rainures/nervures 9 du tube 6. La longueur de l'aiguille hypodermique est telle que, dans la position de stockage de l'aiguille dans la chambre 4 et le tube 6, l'aiguille soit engagée dans le tube 6, avec son extrémité acérée à proximité de la membrane ou paroi ruptible 7. Après mise en place de l'aiguille dans la chambre 4 et le tube 6, la chambre 4 est obturée par un papier pelable et perméable à la vapeur 23, fixé par soudage thermique.

L'utilisation de cette ampoule est la suivante.

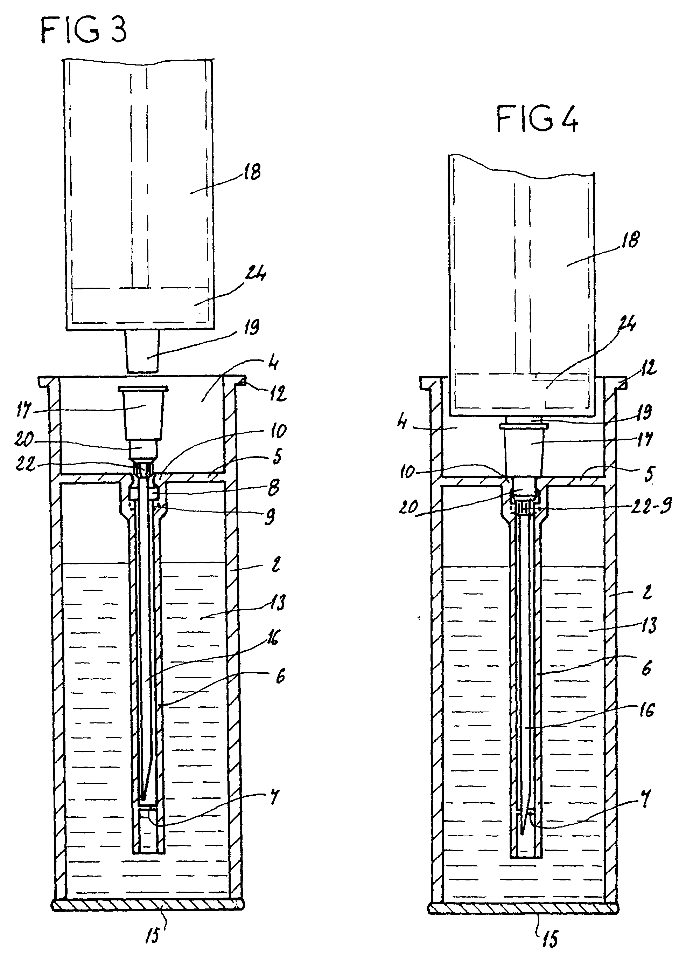

L'opérateur retire tout d'abord le papier 23 pour avoir accès à l'aiguille. Il positionne le corps de la seringue 18 pour introduire le raccord mâle 19 dans le raccord femelle 17 de l'aiguille, le piston 24 de la seringue étant en position avancée. La pression exercée par la seringue sur l'aiguille provoque une translation de l'aiguille qui perfore la membrane 7. Le mouvement de l'aiguille se trouve limité par appui de la partie élargie 17 contre la cloison 5. Dans cette position, le jonc annulaire 10 entoure avec étanchéité la partie 20 de l'aiguille, et les rainures/nervures 22 de l'aiguille sont engagées dans le système rainures/nervures 9 du tube 6. L'aiguille se trouve ainsi bloquée en translation et en rotation, ce qui permet, par un mouvement de rotation, de réaliser un accouplement entre le corps de la seringue et l'aiguille. Dans cette position, l'extrémité acérée de l'aiguille a traversé la paroi 7, mais demeure à l'intérieur du tube 6, ce qui évite tout risque de perforation de l'ampoule. Cette position est représentée à la figure 4.

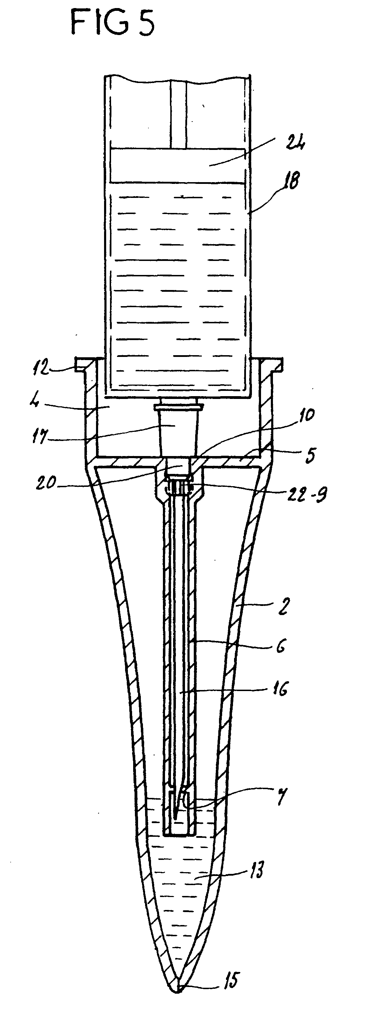

L'opérateur exerce alors une traction sur le piston 24, réalisant l'aspiration de liquide à l'intérieur du corps de la seringue 8. Cette aspiration est rendue possible du fait de la flexibilité du matériau constitutif du corps tubulaire 2, comme montré à la figure 5 qui est une vue en coupe décalée de 90° par rapport aux vues précédentes. Après que la quantité souhaitée de liquide ait été transférée dans la seringue, celle-ci peut être utilisée de façon traditionnelle. Après utilisation de la seringue, l'aiguille peut être replacée à l'intérieur du tube 6, qui joue alors un rôle de protecteur d'aiguille avant destruction de cette dernière.

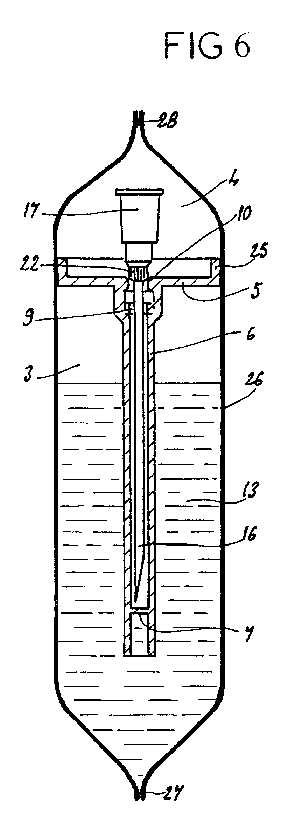

La figure 6 représente une autre forme d'exécution de l'ampoule selon l'invention, dans laquelle les mêmes éléments sont désignés par les mêmes références que précédemment. Dans ce cas, la cloison 5 et le tube 6 sont constitués par une pièce en matière synthétique moulée. La cloison 5 comporte une collerette périphérique 25. Le corps tubulaire 26 est constitué par un tube de matière synthétique extrudée, qui est soudé sur la collerette 25, et qui est fermé par des soudures 27, 28 pour assurer la fermeture respectivement de la chambre 3 et de la chambre 4.

Comme il ressort de ce qui précède, l'invention apporte une grande amélioration à la technique existante en fournissant une ampoule pour le conditionnement d'un liquide à usage médical, servant également au conditionnement de l'aiguille qui sert au transfert du liquide hors de l'ampoule. Cette ampoule possède une structure simple, est d'un prix de revient économique, et procure, compte tenu de ses conditions d'utilisation, une parfaite sécurité tant pour les opérateurs que pour les patients qui seront traités à l'aide du liquide ou d'un produit préparé à partir de ce liquide.

Comme il va de soi, l'invention ne se limite pas aux seules formes d'exécution de cette ampoule, décrites ci-dessus à titre d'exemples, elle en embrasse au contraire toutes les variantes. C'est ainsi notamment que le corps tubulaire en matière synthétique pourrait être obtenu à partir de deux feuilles de matière synthétique éventuellement thermoformées, encore que la cloison entre les deux chambres pourrait ne pas être circulaire, ou que le tube porte-aiguille pourrait ne pas être centré, sans que l'on sorte pour autant du cadre de l'invention.

## Revendications

1. Ampoule pour le conditionnement d'un liquide à usage médical, qui comporte un corps tubulaire (2) en matière synthétique présentant deux chambres isolées de manière étanche par une membrane perforable (7) ou une paroi amovible ou ruptible, dont l'une (3) contient le liquide et dont l'autre (4) contient une aiguille hypodermique (16) dont la partie pointue est tournée vers la membrane perforable ou la paroi amovible ou ruptible et dont l'autre extrémité est munie de moyens de raccordement (17) à un corps de seringue, la chambre (4) contenant l'aiguille (16) comprenant un tube (6) formant porte-aiguille fermé par la membrane (7) ou la paroi amovible ou ruptible et présentant, à son extrémité débouchant dans la chambre contenant l'aiguille, un jonc annulaire (10) d'étanchéité, la longueur du tube (6) destiné à recevoir l'aiguille (16) étant au moins égale à celle de l'aiguille, la membrane (7) ou la paroi amovible ou ruptible étant située à proximité de l'extrémité du tube tournée vers la chambre (3) contenant le liquide et le jonc (10) situé à proximité de l'autre extrémité du tube étant destiné à assurer l'étanchéité autour des moyens de raccordement (17, 20) de l'aiguille (16) à un corps de seringue (18), **caractérisée en ce que** la distance entre l'extrémité libre du tube (6) et la membrane (7) ou la paroi amovible ou ruptible est au moins égale à la course de l'aiguille (16) entre une position de stockage et une position de prélèvement du liquide, dans laquelle l'extrémité pointue de l'aiguille (16) traverse la membrane (7) ou la paroi amovible ou ruptible et les moyens de raccordement (17, 20) de l'aiguille sont enserrés dans le jonc d'étanchéité.

2. Ampoule selon la revendication 1, **caractérisée en ce que** le tube (6) comprend, à proximité du jonc d'étanchéité (10), des moyens (9) de blocage en rotation de l'aiguille (16), tels que nervures ou bossages, destinés à coopérer avec des moyens complémentaires (22) que comporte l'aiguille (16).

3. Ampoule selon l'une quelconque des revendications 1 ou 2, **caractérisée en ce que** le tube (6) destiné à recevoir l'aiguille (16) est solidaire d'une paroi transversale (5) dont la périphérie est solidaire de la paroi intérieure du corps tubulaire (2).

4. Ampoule selon l'une quelconque des revendications 1 à 3, **caractérisée en ce que** le corps tubulaire (2) est réalisé en un matériau souple permettant sa déformation lors de l'aspiration de liquide.

5. Ampoule selon l'une quelconque des revendications 1 à 4, **caractérisée en ce que** le corps tubulaire (2), la paroi (5) séparant les deux chambres et le porte-aiguille (6) sont réalisés en une pièce de matière synthétique obtenue par moulage.

6. Ampoule selon la revendication 5, **caractérisée en ce que** la chambre (3) contenant le liquide est obturée par une soudure thermique, après remplissage en liquide et aplatissement du corps, et la chambre (4) contenant l'aiguille, dont le bord est limité par une collerette (12), est fermée par un opercule (23), tel qu'un film perméable à la vapeur, fixé par soudage à chaud, ou par un obturateur en matière synthétique fixé avec étanchéité par vissage ou encliquetage.

7. Ampoule selon l'une quelconque des revendications 1 à 4, **caractérisée en ce que**, d'une part, la paroi (5) séparant les deux chambres et le porte-aiguille (6) sont réalisés en une pièce de matière synthétique obtenue par moulage et, d'autre part, le corps tubulaire (26) est constitué par un tube de matière synthétique extrudé ou réalisé à partir de deux films de matière synthétique éventuellement thermoformés, fixé par soudage sur la paroi séparant les deux chambres, et dont les deux extrémités sont fermées par des soudures thermiques (27, 28) après formation des deux chambres, remplissage de la chambre (3) contenant le liquide et mise en place de l'aiguille dans l'autre chambre (4).

## Patentansprüche

1. Ampulle zur Aufbewahrung einer Flüssigkeit für den medizinischen Gebrauch, umfassend einen rohrförmigen Körper (2) aus Kunststoff mit zwei Kammern, welche durch eine durchbdringbare Membran (7) oder durch eine herausziehbare oder zerbrechbare Wand dicht abgetrennt sind, wobei die eine Kammer (3) die Flüssigkeit enthält und die andere Kammer (4) eine Injektionsnadel (16) enthält, deren Spitze auf die durchdringbare Membran oder die herausziehbare oder zerbrechbare Wand zu weist und deren anderes Ende mit Verbindungsmitteln (17) für einen Spritzenkörper versehen ist, wobei die die Nadel (16) enthaltende Kammer (4) ein einen Nadelträger bildendes Rohr (6) umfasst, das durch die Membran (7) oder die herausziehbare oder zerbrechbare Wand verschlossen ist und an seinem in die die Nadel enthaltende Kammer mündenden Ende einen Dichtungsring (10) aufweist, wobei die Länge des zur Aufnahme der Nadel (16) bestimmten Rohrs (6) wenigstens gleich jener der Nadel ist, wobei die Membran (7) oder die herausziehbare oder zerbrechbare Wand in der Nähe des Endes des Rohrs angeordnet ist, welches auf die die Flüssigkeit enthaltenden Kammer (3) zu gerichtet ist, und der in der Nähe des anderen Endes des Rohrs angeordnete Ring (10) dazu bestimmt ist, die Dichtigkeit um die Verbindungsmittel (17, 20) der Nadel (16) mit einem Spritzenkörper (18) herum sicherzustellen, **dadurch gekennzeichnet, dass** der Abstand zwischen dem freien Ende des Rohrs (6) und der Membran (7) oder der herausziehbaren oder zerbrechbaren Wand wenigstens gleich dem Weg der Nadel (16) zwischen einer Lagerungsposition und einer Flüssigkeitsentnahmeposition ist, in welcher das spitze Ende der Nadel (16) die Membran (7) oder die herausziehbare oder zerbrechbare Wand durchdringt und die Verbindungsmittel (17, 20) der Nadel von dem Dichtungsring umfasst sind.

2. Ampulle nach Anspruch 1, **dadurch gekennzeichnet, dass** das Rohr (6) in der Nähe des Dichtungsrings (10) Mittel (9) zum Drehblockieren der Nadel (16) umfasst, beispielsweise Rippen oder Wülste, welche dazu bestimmt sind, mit komplementären Mitteln (22) zusammenzuwirken, welche die Nadel (16) umfasst.

3. Ampulle nach einem der Ansprüche 1 oder 2, **dadurch gekennzeichnet, dass** das zur Aufnahme der Nadel (16) bestimmte Rohr (6) mit einer Querwand (5) verbunden ist, deren Umfang mit der Innenwand des rohrförmigen Körpers (2) verbunden ist.

4. Ampulle nach einem der Ansprüche 1 bis 3, **dadurch gekennzeichnet, dass** der rohrförmige Körper (2) aus einem geschmeidigen Material gebildet ist, welches seine Verformung beim Einsaugen der Flüssigkeit zulässt.

5. Ampulle nach einem der Ansprüche 1 bis 4, **dadurch gekennzeichnet, dass** der rohrförmige Körper (2), die die beiden Kammern trennende Wand (5) und der Nadelträger (6) aus einem gegossenen Kunststoffstück gebildet sind.

6. Ampulle nach Anspruch 5, **dadurch gekennzeichnet, dass** die die Flüssigkeit enthaltende Kammer (3) nach Einfüllen der Flüssigkeit und Glätten des Körpers durch thermische Verschweißung verschlossen ist, und die die Nadel enthaltende Kammer (4), deren Rand von einem Kragen (12) begrenzt ist, durch einen Deckel (23) verschlossen ist, beispielsweise einen dampfdurchlässigen Film, das durch Warmschweißen befestigt ist, oder von einem Verschluss aus Kunststoff, der durch Verschrauben oder Verrasten dicht befestigt ist.

7. Ampulle nach einem der Ansprüche 1 bis 4, **dadurch gekennzeichnet, dass** einerseits die die beiden Kammern trennende Wand (5) und der Nadelträger (6) aus einem gegossenen Kunststoffstück gebildet sind, und andererseits der rohrförmige Körper (26) von einem Kunststoffrohr gebildet ist, das extrudiert oder aus zwei gegebenenfalls thermogeformten Kunststofffilmen gebildet und durch Schweißen an der die beiden Kammern trennenden Wand befestigt ist, und dessen beide Enden nach Bildung der beiden Kammern, Befüllen der die Flüssigkeit enthaltenden Kammer (3) und Anordnen der Nadel in der anderen Kammer (4) durch thermisches Verschweißen (27, 28) geschlossen sind.

## Claims

1. Ampoule for packaging a liquid for medical use, which comprises a tubular body (2) made of a synthetic material having two chambers isolated in a leakproof manner by a membrane that can be perforated (7) or a detachable or breakable wall, one chamber (3) of which contains the liquid and the other chamber (4) of which contains a hypodermic needle (16) of which the pointed part is turned towards the membrane that can be perforated or the detachable or breakable wall and of which the other end is provided with means of connection (17) to a syringe body, the chamber (4) containing the needle (16) comprising a tube (6) forming a needle-carrier closed by the membrane (7) or the detachable or breakable wall and having, at its end emerging into the chamber containing the needle, an annular retaining ring (10), the length of the tube (6) designed to receive the needle (16) being at least equal to that of the needle, the membrane (7) or the detachable or breakable wall being situated close to the end of the tube turned towards the chamber (3) containing the liquid and the retaining ring (10) situated close to the other end of the tube being designed to ensure leakproofness about the means of connection (17,20) of the needle (16) to the syringe body (18), **characterized in that** the distance between the free end of the tube (6) and the membrane (7) or the detachable or breakable wall is at least equal to the traverse of the needle (16) between a storage position and a position for drawing up liquid, in which the pointed end of the needle (16) passes through the membrane (7) or the detachable or breakable wall and the means (17,20) for connecting the needle are gripped in the leakproof retaining ring.

2. Ampoule according to claim 1, **characterized in that** the tube (6) comprises, close to the retaining ring (10), means (9) for locking the needle (16) in rotation, such as ribs or projections designed to cooperate with the complementary means (22) carried by the needle (16).

3. Ampoule according to either of claims 1 or 2, **characterized in that** the tube (6) designed to receive the needle (16) is secured to a transverse wall (5) of which the periphery is secured to the interior wall of the tubular body (2).

4. Ampoule according to any one.of claims 1 to 3, **characterized in that** the tubular body (2) is made of a flexible material enabling it to deform during aspiration of liquid.

5. Ampoule according to any one of claims 1 to 4, **characterized in that** the tubular body (2), the wall (5) separating the two chambers and the needle-carrier (6) are made in one piece of a synthetic material obtained by moulding.

6. Ampoule according to claim 5, **characterized in that** the chamber (3) containing the liquid is closed by a hot weld, after filling the body with liquid and flattening it, and the chamber (4) containing the needle, of which the edge is limited by a collar (12), is closed by cap (23), such as a vapour-permeable film, attached by hot welding, or by a stopper made of synthetic material attached in a leakproof manner by screwing or snapping-on.

7. Ampoule according to any one of claims 1 to 4, **characterized in that**, on the one hand, the wall (5) separating the two chambers and the needle-carrier (6) are made in one piece of synthetic material obtained by moulding and, on the other hand, the tubular body (26) consists of a tube made of extruded synthetic material or is made from two films of synthetic material that may be thermoformed, attached by welding onto the walls separating the two chambers, and of which the two ends are closed by hot-welds (27,28) after forming the two chambers, filling the chamber (3) containing the liquid and putting the needle in place in the other chamber (40).
